(19) Europäisches Patentamt
European Patent Office
Office européen des brevets

(11) Numéro de publication: **0 161 987**
**B1**

(12) **FASCICULE DE BREVET EUROPÉEN**

(45) Date de publication du fascicule du brevet: **23.11.88**

(21) Numéro de dépôt: **85400876.0**

(22) Date de dépôt: **06.05.85**

(51) Int. Cl.⁴: **C 07 C 121/66**, C 07 D 215/10, C 07 D 213/20, C 07 D 215/20, C 07 D 277/10, C 07 D 213/79, C 07 D 209/08, C 07 D 471/04 // (C07D471/04, 221:00, 221:00)

(54) **Complexes organiques amphiphiles a transfert de charge, conducteurs de l'électricité ou precurseurs de conducteurs de l'électricité.**

(30) Priorité: **10.05.84 FR 8407212**

(43) Date de publication de la demande: **21.11.85 Bulletin 85/47**

(45) Mention de la délivrance du brevet: **23.11.88 Bulletin 88/47**

(84) Etats contractants désignés: **DE FR GB NL**

(73) Titulaire: **COMMISSARIAT A L'ENERGIE ATOMIQUE, 31/33, rue de la Fédération, F-75015 Paris (FR)**

(72) Inventeur: **Barraud, André, 4, rue des Closeaux, F-91440 Bures sur Yvette (FR)**
Inventeur: **Ruaudel, Annie, 14, Allée des Frondaisons, F-91370 Verrieres Le Buisson (FR)**

(74) Mandataire: **Mongrédien, André, c/o BREVATOME 25, rue de Ponthieu, F-75008 Paris (FR)**

(56) Documents cité:
**FR-A-2 375 623**
**US-A-3 681 353**

**CHEMICAL ABSTRACTS, vol. 75, no. 24, 13 décembre 1971, page 8, no. 141281c, Columbus, Ohio, US; A. REMBAUM et al.: "Electrical properties of 7,7',8,8'-tetracyanoquinodimethan salts of ionene polymers and their model compounds"**
**JOURNAL OF THE AMERICAN CHEMICAL SOCIETY, vol. 93, no. 10, 19 mai 1971, pages 2532-2534; A. REMBAUM et al.: "Electron transfer to bipyridilium (paraquat) salts"**
**CHEMISCHE BERICHTE, vol. 115, no. 7, 1982, pages 2643-2651, Verlag Chemie, Weinheim, DE; J. DAUB et al.: "Komplexbildung und Elektronentransferreaktionen von 8,8-Bis-(dimethylamino)heptafulven 1), Umsetzungen mit**

(56) Documents cité: (suite)
**Nonacarbonyldieisen und Tetracyanchinodimethan"**
**BULLETIN OF THE CHEMICAL SOCIETY OF JAPAN, vol. 51, no. 5, mai 1978, pages 1311-1314; S. ARAKI et al.: "N,N-Disubstituted 1,3-dithiolan-2-iminium-TCNQ- salts: Effect of the substituents on the nitrogen atom on the composition and the electrical conductivity"**

**EP 0 161 987 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention a pour objet de nouveaux complexes organiques amphiphiles à transfert de charge, conducteurs de l'électricité ou précurseurs de conducteurs de l'électricité.

On rappelle que les complexes à transfert de charge sont formés par l'association de deux molécules dont l'une, A, agit comme accepteur d'électrons et l'autre, D, agit comme donneur d'électrons. On peut représenter cette réaction de la façon suivante:

$$mA + nD \rightarrow A_m^{-\rho} D_n^{+\rho}$$

m et n représentant les nombres respectifs de molécules et p le transfert de charge correspondant à la proportion d'électrons transférés.

Si m = n, on a une stoechiométrie simple et si m est différent de n, on a une stoechiométrie complexe.

Si ρ est sensiblement égal à 0, le complexe est un complexe moléculaire à état fondamental neutre.

Si ρ est égal à 1, c'est un vrai composé ionique avec une charge électrique par molécule D ou A, et si P est inférieur à 1, il y a moins de charges électriques que de molécules présentes. On a ainsi un composé à valence mixte, condition nécessaire oour obtenir un conducteur organique. On peut alors distinguer:

1) - les vrais complexes à transfert de charge dans lesquels il y a transfert d'un électron du donneur à l'accepteur; ces électrons célibataires se trouvent alors sur des orbitales π (complexe π-π); et
2) - les sels d'ions radicalaires dans lesquels un seul des ions est radicalaire, le contre-ion étant diamagnétique.

Des complexes de ce type sont en particulier décrits dans: Annales de Physique, 1976, tome 1, n° 4 - 5, pages 145 à 256 et dans Journal de Chinie Physique, 1982, 79, n° 4.

Le document Bulletin of Chemical Society of Japan, Col 51(5), 1311 - 1314, 1978, décrit des composés de ce type qui sont des sels de tétracyanoquinodiméthane (TCNQ) dans lesquels le groupe donneur d'électrons comporte des chaînes hydrocarbonées ayant 6, 8 ou 10 atomes de carbone.

Les articles du Journal de Physique, colloque n° 3, supplément au n° 6, tome 44, juin 1983, pages 1307 à 1312 et pages 1261 à 1264, illustrent également des composés de ce type dans lesquels l'accepteur d'électrons est le tétracyanoquinodiméthane (TCNQ). Dans ces articles, on a étudié l'influence de différents substituants sur les propriétés électriques du complexe, et on a ainsi pu constater que la présence de substituants encombrants comportant plusieurs atomes de carbone et/ou des cycles était néfaste, car elle diminuait de façon importante la conductivité électrique du complexe.

Ainsi, pour cette raison, il n'a jamais été envisagé jusqu'à présent de réaliser des complexes à transfert de charge comportant des substituants hydrocarbonés ayant au moins 12 atomes de carbone. Or, la présence de tels substituants permet de conférer à la molécule un caractère amphiphile qui peut être intéressant pour certaines applications.

On rappelle que des molécules "amphiphiles" ou "amphipathiques" sont des molécules organiques possédant une partie hydrophobe, c'est-à-dire une partie ayant une répulsion pour les liquides polaires tels que l'eau et une partie hydrophile, c'est-à-dire une partie ayant une affinité pour les liquides polaires tels que l'eau.

Grâce à ces caractéristiques particulières, lorsque l'on introduit des molécules amphiphiles sur la surface d'un liquide tel que l'eau les molécules s'étalent à la surface du liquide et elles s'orientent de façon que leur partie hydrophile plonge dans l'eau, tandis que leur partie hydrophobe généralement constituée par une chaîne hydrocarbonée, tend à s'en écarter de sorte que l'axe de la chaîne devient perpendiculaire à la surface de l'eau. Si les forces de cohésion entre molécules sont suffisantes, ces molécules restent groupées et limitent leur étalement à un film monomoléculaire continu ayant sensiblement l'épaisseur d'une molécule, ce qui correspond à un film de Langmuir. De tels films peuvent être manipulés sur la surface de l'eau, puis déposés sur un support solide après avoir été comprimés sous une pression superficielle appropriée, selon la méthode connue de Langmuir Blodgett décrite dans J. of Am. Chem. Soc., vol. 57 (1935) p. 1007 - 1010.

On peut utiliser cette méthode pour déposer sur un support, plusieurs couches monomoléculaires de même nature ou de natures differentes. Dans ce cas, les molécules de chacune des couches ont toutes la même orientation; et des films constitués par un ensemble de telles couches trouvent des applications intéressantes en électricité, en électronique, etc... en raison de leur structure particulière et de leur organisation.

Lorsque les molécules sont des complexes organiques à transfert de charge, conducteurs de l'électricité, la réalisation de tels films présente de l'intérêt car ces films peuvent être utilisés dans des dispositifs détecteurs de gaz, de températures ou de composants d'une solution, dont le fonctionnement est basé sur la modification de l'une des propriétés électriques du complexe en fonction de l'influence d'un agent extérieur tel que la température, la présence d'un gaz, etc...

Par ailleurs, de tels films présentent une anisotropie de conductivité considérable qui les rend particulièrement intéressants.

De plus, des complexes à transfert de charge présentant la propriété d'être amphiphiles peuvent être utilisés comme charge conductrice dans des peintures ou dans des émulsions.

La présente invention a précisément pour objet des complexes organiques à transfert de charge conducteurs

de l'électricité ou précurseurs de conducteurs de l'électricité, qui présentent justement la propriété d'être amphiphiles.

Ces complexes amphiphiles à transfert de charge répondent à la formule:

$$DA_xX_y \qquad (I)$$

dans laquelle D représente un groupe organique donneur d'électrons monomérique, A représente un groupe organique accepteur d'électrons, X représente un accepteur d'électrons non amphiphile choisi parmi les acides de Lewis, x est un nombre supérieur à 0 et y est égal à 0 ou est un nombre supérieur à 0, l'un au moins des groupes D et A étant amphiphile et comportant au moins un substituant hydrocarboné saturé ou insaturé d'au moins 12 atomes de carbone.

Généralement, x et y peuvent prendre des valeurs allant jusqu'à 20.

De préférence, le substituant hydrocarboné a de 14 à 30 atomes de carbone.

Dans la formule précitée, X représente avantageusement un acide de Lewis choisi parmi $PF^-_6$, $ClO^-_4$, $BF^-_4$, $ReO^-_4$, $IO^-_4$, $FSO^-_3$, $AsF^-_6$, $AsF^-_4$, $Br^-$, $Cl^-$, $MnCl^-_6$, les iodures et les oxydes nitreux et nitriques.

De préférence, lorsque le complexe comprend un accepteur d'électrons non amphiphile, c'est-à-dire lorsque y est différent de 0, cet accepteur d'électrons non amphiphile est $I^-$.

Selon un mode préféré de réalisation de l'invention, le groupe organique donneur d'électrons monomérique D est un groupe amphiphile comportant au moins un substituant hydrocarboné ayant au moins 12 atomes de carbone. Dans ce cas, D est avantageusement une base aliphatique, aromatique ou hétérocyclique comportant au moins un substituant hydrocarboné, saturé ou insaturé ayant au moins 12 atomes de carbone.

Parmi les bases aliphatiques susceptibles d'être utilisées, on peut citer les groupes ammonium quaternaire de formule:

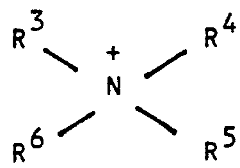

dans laquelle $R^3$, $R^4$, $R^5$ et $R^6$ qui peuvent être identiques ou différents sont des radicaux alkyle ou des chaînes hydrocarbonées comportant éventuellement une ou plusieurs doubles et/ou triples liaisons, l'un au moins des groupes $R^3$, $R^4$, $R^5$ et $R^6$ ayant au moins 12 atomes de carbone.

Les bases aromatiques susceptibles d'être utilisées peuvent être avantageusement constituées par des groupes ammonium quaternaire dérivés de l'aniline ou de dérivés substitués de l'aniline.

Les bases hétérocycliques susceptibles d'être utilisées peuvent être constituées par des groupements ammonium quaternaire dérivés des pyridines, des pipéridines, des bipyridines, des benzopyridines par exemple des quinoléines, des isoquinoléines, des acridines, des phénazines et des phénanthrolines. Ces groupements peuvent répondre en particulier aux formules suivantes:

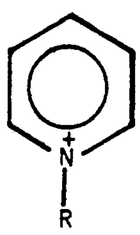 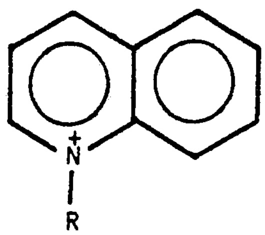 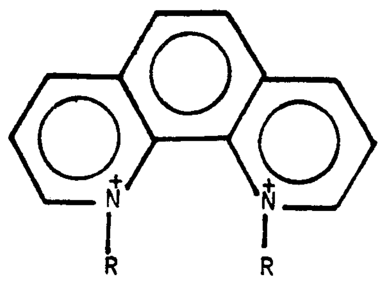

dans lesquelles le substituant R de l'atome d'azote peut être le radical hydrocarboné ayant plus de 12 atomes de carbone. Ce substituant R peut être également un atome d'hydrogène ou un radical alkyle inférieur ayant par exemple 1 à 4 atomes de carbone. Dans ce cas, le ou les noyau(x) benzénique(s) ou hétérocyclique(s) comporte(nt) au moins un autre substituant constitué oar un radical hydrocarboné ayant plus de 12 atomes de carbone.

Ces noyaux peuvent également comporter un ou plusieurs autres substituants tels que des radicaux alkoxy, des radicaux alkyle, des radicaux COOR avec R représentant un radical alkyle, des atomes d'halogène, etc...

Le groupe D peut aussi représenter une base hétérocyclique comportant plusieurs hétéro-atomes de nature différente, par exemple des atomes d'azote et de soufre. A titre d'exemple de telles bases hétérocycliques, on peut citer les N-alkylbenzothiazoles et leurs dérivés substitués, et les N-alkylindoléniniumtriméthinecyanines et leurs dérivés substitués.

On précise que selon l'invention, le terme "radical hydrocarboné saturé ou insaturé" désigne des radicaux formés d'atomes de carbone et d'hydrogène comportant éventuellement une ou plusieurs doubles et/ou triples

liaisons. Ceux-ci peuvent être reliés au groupe accepteur d'électrons A et/ou au groupe donneur d'électrons D par une simple liaison, un atome d'oxygène ou un groupe -COO-.

Dans le complexe amphiphile de l'invention, le groupe organique accepteur d'électrons A peut être choisi parmi le tétracyanoéthylène (TCNE), l'hexacyanobutadiène (HCBD), le 1,2,4,5-tétracyanobenzène (TCNB), le 7,7,8,8,-tétracyanoquinodiméthane (TCNQ) et ses dérivés substitués, le tétracyanodiquinodiméthane (TCNDQ), le benzotétracyanoquinodiméthane (benzo-TCNQ), le 2,3,5,6,-tétrachloro-p-benzoquinone (CA), le trinitrobenzène (TNB), le tétranaphtoquinodiméthane (TNAP), le thiophènetétracyanoquinodiméthane (thiophène-TCNQ), le sélénophène-tétracyanoquinodiméthane (sélénophène-TCNQ), et le tétracyanoquinoquinazolino quinazoline (TCQQ).

On peut également utiliser dans l'invention les dérivés substitués de ces groupes accepteurs d'électrons, par exemple des dérivés halogénés tels que les dérivés fluorés, des dérivés alcoxylés et des dérivés alkylés.

Selon un mode préféré de réalisation de l'invention, l'accepteur d'électrons A répond à la formule:

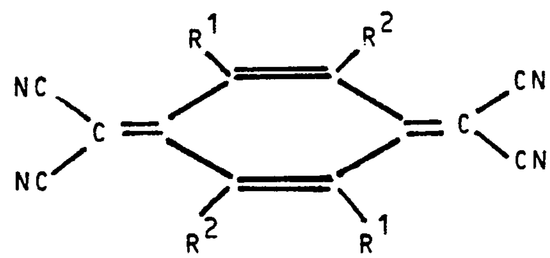

(II)

dans laquelle $R^1$ et $R^2$, qui peuvent être identiques ou différents représentent un atome d'hydrogène, de chlore, de fluor ou de brome, ou un radical alkyle.

De préférence encore, l'accepteur d'électrons A est le tétracyanoquinodiméthane, soit le dérivé de formule (II) dans laquelle $R^1$ et $R^2$ représentent un atome d'hydrogène.

Les complexes de l'invention peuvent être obtenus sous la forme de conducteurs de l'électricité ou de précurseurs de conducteurs de l'électricité. Dans ce dernier cas, il est nécessaire de les soumettre ensuite à un traitement physique et/ou à un traitement chimique pour les transformer en conducteurs de l'électricité.

Ces complexes sont généralement obtenus sous la forme de poudres ou de cristaux de couleur sombre, et ils se caractérisent par des propriétés optiques particulières dans l'infrarouge et, pour certains d'entre eux, par une conduction de l'électricité. Ainsi, les complexes de formule (I) dans laquelle y est supérieur à 0 sont conducteurs et présentent un spectre d'absorption infrarouge caractérisé par une très large bande d'absorption électronique entre 1 micron et 10 microns environ (parfois même plus large) et par un élargissement très important des bandes d'absorption vibrationnelle des groupements impliqués dans le transfert de charge (par exemple les bandes à 1100 et 1300 $cm^{-1}$ dans le cas où le groupe A du complexe est du tétracyanoquinodiméthane (TCNQ)).. Les complexes de formule (I) dans laquelle x est différent de 1 et y est égal à 0, sont également conducteurs de l'électricité et présentent des spectres infrarouges assez comparables.

En revanche, les complexes de formule (I) dans laquelle $x=1$ et $y=0$ sont isolants et présentent un renforcement marqué des bandes vibrationnelles relatives aux groupements impliqués dans le transfert de charge, avec des déformations de ces bandes qui, tout en restant étroites, deviennent dissymétriques.

Les résistivités de ces complexes s'échelonnent de $10^1$ à $10^4$ Ω.cm à 20°C pour les complexes amphiphiles conducteurs en poudres et sont supérieures à 10 Ω.cm pour les complexes précurseurs de complexes conducteurs.

Les complexes de l'invention se différencient notamment des complexes à transfert de charge connus actuellement par le fait que leur cristallisation est de type smectique, c'est-à-dire que chaque plan conducteur est mono(ou bi) moléculaire et est découplé du suivant par le plan isolant de la partie hydrophobe du complexe amphiphile. Ces matériaux conducteurs sont donc essentiellement unidimensionnels avec un caractère bidimensionnel partiel, alors que les complexes à transfert de charge connus actuellement sont des conducteurs monodimensionnels partiellement tridimensionnels. Ceci confère aux conducteurs de l'invention sous la forme de monocristaux une anisotropie de conductivité considérable.

En raison de leur caractère amphiphile, les complexes à transfert de charge de l'invention peuvent être mis sous la forme de couches monomoléculaires par la méthode de Langmuir Blodgett comme on l'a vu précédemment.

Les complexes à transfert de charge de l'invention peuvent être préparés par des procédés classiques tels que celui décrit par L.R. Melby et al. dans Journ. of Am. Chem. Soc., vol. 84 (1962), p. 3374 - 3387.

Lorsque l'on veut préparer des complexes à transfert de charge de formule (I) dans laquelle $y=n$, soit des complexes de formule $DA_x$, on fait réagir un halogénure du groupe organique donneur d'électrons D avec un sel du groupe accepteur d'électrons A, par exemple l'iodure de D avec le sel de lithium de A. On opère généralement dans un solvant polaire tel que l'alcool éthylique, l'acétonitrile, etc...

Lorsque l'on veut préparer des complexes de formule (I) dans laquelle y est différent de 0, on fait réagir un composé de D et de X (obtenu en combinant l'acide de Lewis X avec le grouoe organique donneur d'électrons

D) sur le groupe organique accepteur d'électrons A. Dans le cas où le groupe X représente l'iode, on fait réagir l'iodure du groupe donneur d'électrons D sur le groupe accepteur d'électrons.

Les composés de départ utilisés pour la préparation de ces complexes, par exemple les bases organiques substituées comportant une longue chaîne hydrocarbonée, sont obtenus par des procédés classiques. De même, les groupements accepteurs d'électrons A sont des composés connus qui sont obtenus également par des procédés classiques.

A titre d'exemple de complexes de l'invention repondant à la formule (I) avec y=0, on peut citer:

- les complexes de formule: $D(TCNQ)_x$ dans laquelle x est égal à 1 ou 1,5 et D représente:

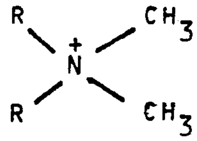 ; 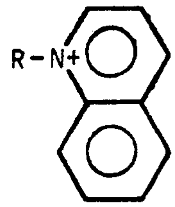 ; 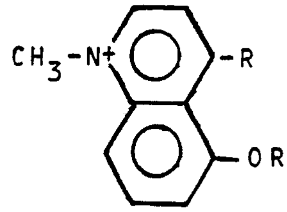 ;

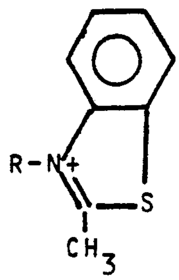

avec R représentant un groupe alkyle ayant 12 à 30 atomes de carbone.
- les complexes de formule $D(TCNQ)_2$ avec D représentant:

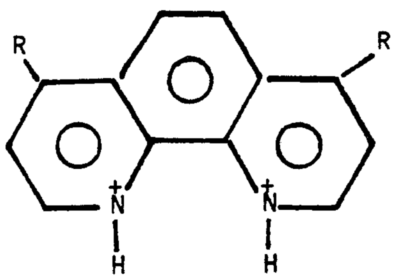

et R étant un radical alkyle ayant 12 à 30 atomes de carbone.
- les complexes de formule D(TCNQ) avec D représentant:

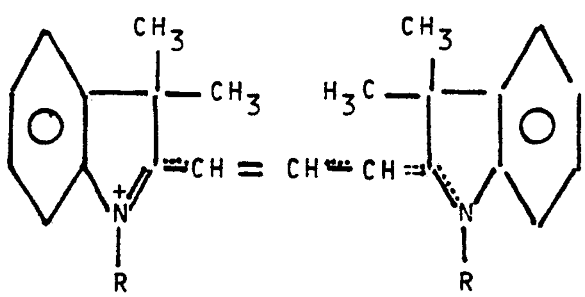

et R étant un radical alkyle ayant 12 à 30 atomes de carbone.

A titre d'exemple de complexes de l'invention répondant à la formule (I) avec y > 0, on peut citer les complexes de formule:

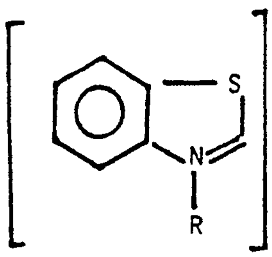 $(TCNQ)_{0,5}$ I ; 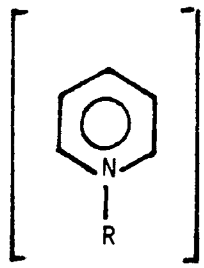 $(TCNQ)_{2/3}$ $I_3$ et

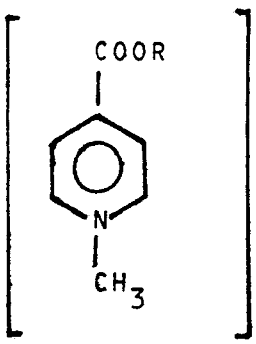 $(TCNQ)_{2/3}$ $I_3$

dans lesquelles R représente un groupe alkyle de 12 à 30 atomes de carbone.

Les exemples suivants sont donnés bien entendu à titre non limitatif pour illustrer la réalisation de complexes à transfert de charge conformes à l'invention.

Dans tous ces exemples, on a caractérisé les produits obtenus par spectrométire dans les domaines infrarouge et visible, et on a effectué des mesures de résistivité électrique des complexes obtenus sur des pastilles de poudre comprimée à 10 tonnes/cm² ($10^3$ MPa).

**Exemple 1**

On fait réagir à chaud dans une solution d'alcool éthylique 1 mmol d'iodure de dioctadécyl diméthyl ammonium et 1 mmol de sel de lithium du tétracyanoquinodiméthane (TCNQ). On obtient ainsi, après refroidissement de la solution, un précipité de cristaux bleus du complexe de formule:

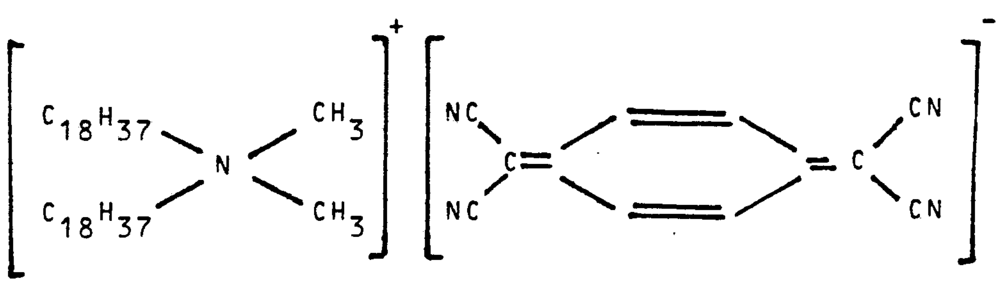

soit d'un complexe de formule (I) dans laquelle D représente le dioctadécyl diméthyl ammonium, A représente TCNQ, x=1 et y=0.

On contrôle la composition de ce complexe par mesure d'absorption dans le domaine visible d'une solution de ce complexe dans l'acétonitrile, et par le spectre d'absorption infrarouge en matrice de kBr.

Le spectre infrarouge indique que ce complexe est un complexe à transfert de charge isolant.

**Exemple 2**

On traite par un excés de TCNQ le complexe obtenu dans l'exemple 1 en solution dans l'acétonitrile. Il précipite à froid un complexe noir de formule:

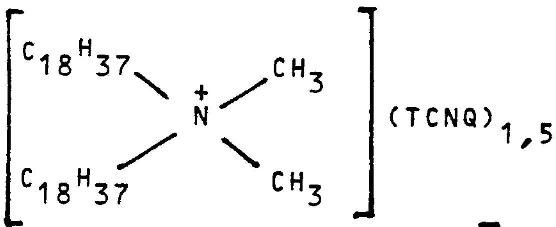

ou

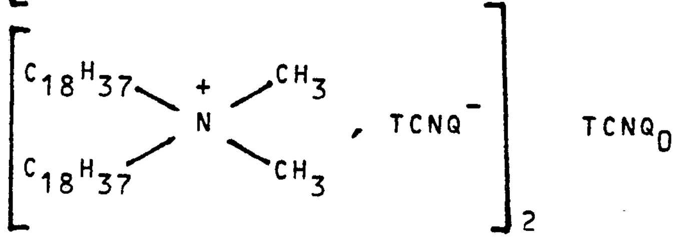

ce qui correspond à un complexe avec x=1,5. On caractérisé ce complexe comme dans l'exemple 1 par absorption dans le visible et dans l'infrarouge. Le spectre d'absorption infrarouge en matrice de kBr indique que ce complexe est semi-conducteur de l'électricité.

**Exemple 3**

On fait réagir en milieu alcoolique une mmol d'iodure de N-docosylquinolinium avec une mmol du sel de lithium du TCNQ. On obtient ainsi un complexe bleu répondant à la formule:

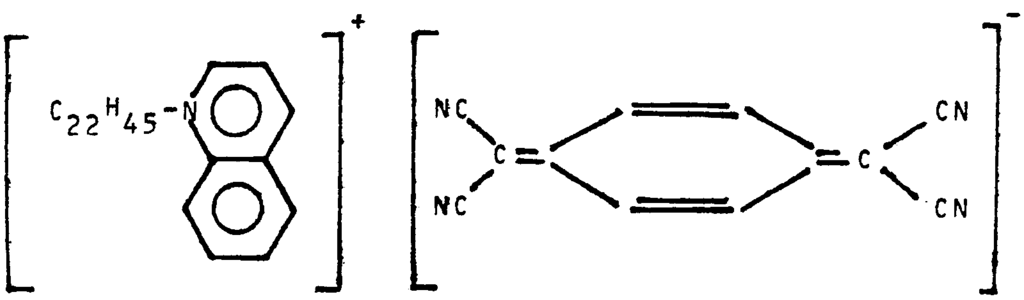

Ce complexe correspond à un complexe de formule (I) dans laquelle D représente le N-docosyl quinolinium, A représente TCNQ, x =1 et y=0. Ce complexe a été défini, caractérisé et dosé par son spectre d'absorption dans les domaines infrarouge et visible.

Des mesures de résistivité effectuées sur une pastille de poudre comprimée sous 10 tonnes/$cm^2$ ($10^3$ MPa) indiquent que ce complexe est isolant.

**Exemple 4**

On dissout le complexe obtenu dans l'exemple 3 dans de l'acétonitrile et on fait réagir celui-ci avec un excés de TCNQ. Il cristallise au refroidissement un autre complexe bleu-noir de même formule que celui de l'exemple 3 sauf que x=1,5 au lieu de 1. Ce complexe est caractérisé comme celui de l'exemple 3 par spectrométrie d'absorption dans le domaine infrarouge et dans le domaine visible.

Des mesures de résistivité effectuées dans les mêmes conditions montrent que ce complexe est semi-conducteur.

**Exemple 5**

On dissout dans de l'alcool chaud de l'iodure de N-méthyl, 4-octadécyl,5-octadécyloxy quinoléinium et on le fait réagir dans ce milieu avec son équivalent molaire de sel de lithium de TCNQ. Il précipite au refroidissement un complexe bleu vert répondant à la formule:

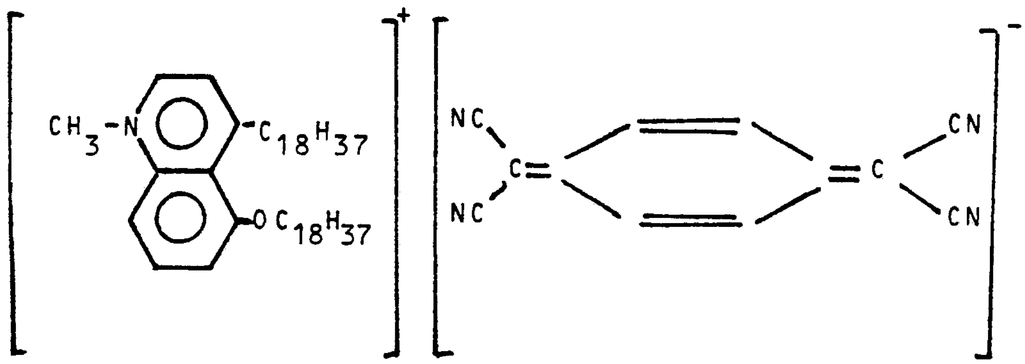

On a ainsi un complexe répondant à la formule (I) avec D représentant le N-méthyl, 4-octadécyl, 8-octodécyloxy quinoléinium, A représentant TCNQ, x=1 et y=0.

La caractérisation de ce complexe a été faite comme dans les exemples 3 et 4, et on a constaté que le complexe était isolant.

**Exemple 6**

On fait réagir un excés de TCNQ dans de l'acétonitrile chaud sur le complexe obtenu dans l'exemple 5 et l'on précipite ainsi un nouveau complexe violet semi-conducteur qui répond à la même formule que le complexe de l'exemple 5, sauf que x=1,5 au lieu de 1. Ce complexe est semi-conducteur.

**Exemple 7**

On fait réagir dans de l'acétonitrile 2 mmol de 4-7-didocosylphénanthroline avec 3 mmol de tétracyanoquinodiméthane et 1 mmol de dihydrotétracyanoquinodiméthane (TCNQ $H_2$). On précipite ainsi un complexe noir répondant à la formule:

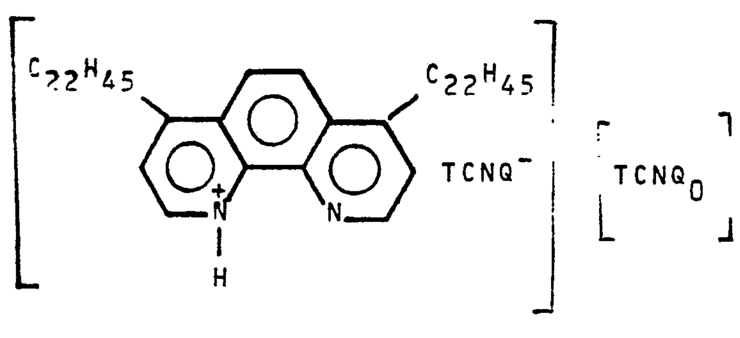

soit un complexe de formule (I) dans laquelle x=2 et y=0.

Ce complexe a été caractérisé comme précédemment par spectrophotométrie dans le domaine infrarouge et dans le domaine visible et des mesures de résistivité ont montré qu'il était conducteur de l'électricité.

**Exemple 8**

On fait réagir dans de l'alcool 0,5 mmol d'iodure de N-eicosyl 3,3'-diméthylindoléninium triméthonecyanine avec 0,5 mmol du sel de lithium de TCNQ. On obtient ainsi un complexe marron rouge de formule:

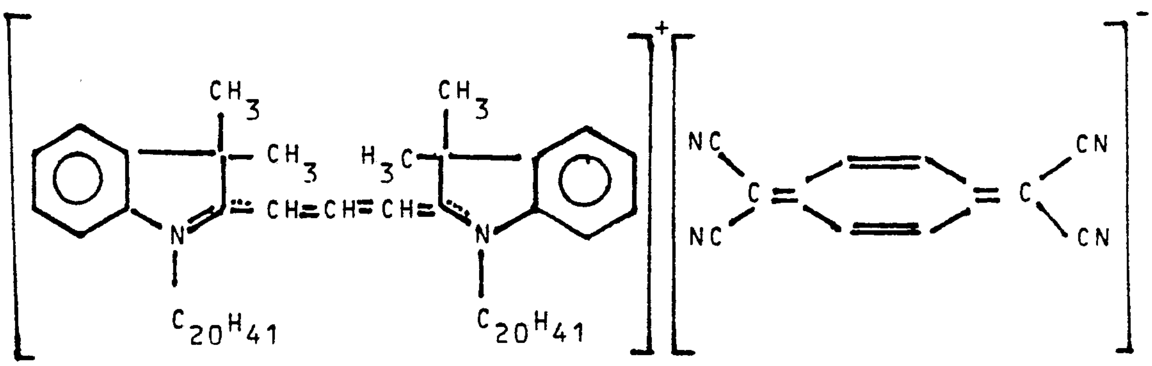

soit un complexe de formule (I) dans laquelle D représente le N-eicosyl 3,3'-diméthyl indoléninium triméthine cyanine, A représente TCNQ, x=1 et y=0.

**Exemple 9**

On fait réagir dans de l'acétonitrile 1 mmol d'iodure de N-octadécyl-benzothiazole avec 0,66 mmol de tétracyanoquinodiméthane. On obtient ainsi un complexe marron répondant à la formule:

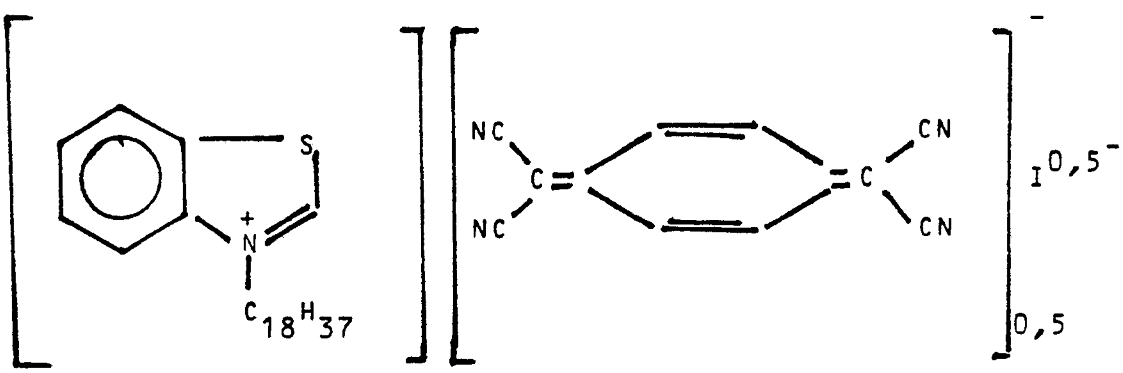

soit un complexe de formule (I) avec D représentant le N-octadécyl benzothiazolium, A représentant TCNQ, X représentant I, x=0,5 et y=1.

Ce complexe a été caractérisé par son spectre dans l'infrarouge et son spectre visible et des mesures de résistivité effectuées sur des pastilles de poudre comprimée à 10 tonnes/$cm^2$ ($10^3$ Mpa) ont montré qu'il était conducteur de l'électricité.

**Exemple 10**

On fait réagir dans de l'acétonitrile, 0,6 mmol de TCNQ avec 1 mmol d'iodure de N-octadécylpyridinium. On obtient ainsi des cristaux noirs d'un complexe répondant à la formule:

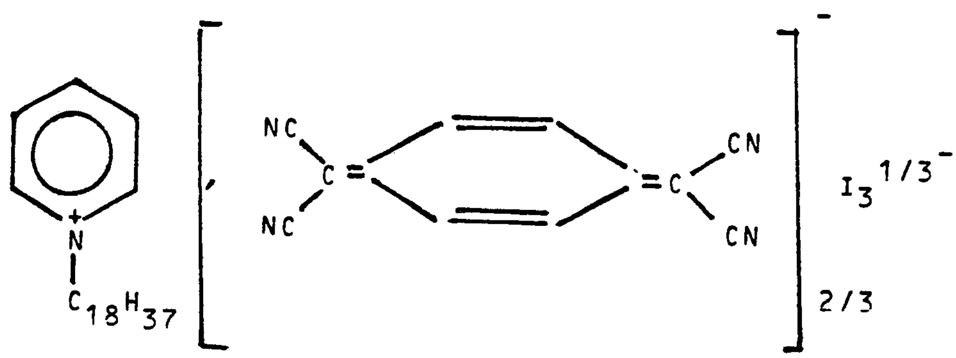

soit un complexe de la forme $DA_xX_y$ avec x=2/3 et y=3.

Ce complexe a été caractérisé par spectrophotométrie dans le visible et dans l'infrarouge et des mesures de résistivité ont montré qu'il était conducteur de l'électricité.

**Exemple 11**

On fait réagir en solution alcoolique chaude 1 mmol d'iodure N-docosyl pyridinium avec 1 mmol du sel de lithium de TCNQ. On obtient ainsi un complexe répondant à la formule:

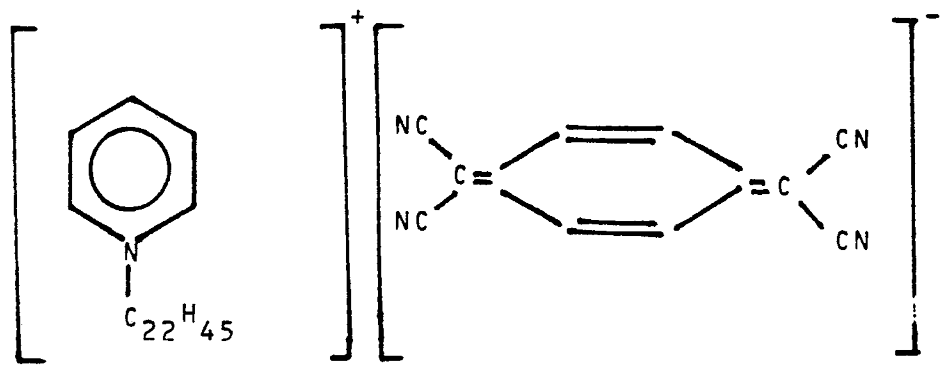

On traite ensuite ce complexe par un excés de TCNQ dans de l'acétonitrile et on cristallise ainsi un complexe de formule:

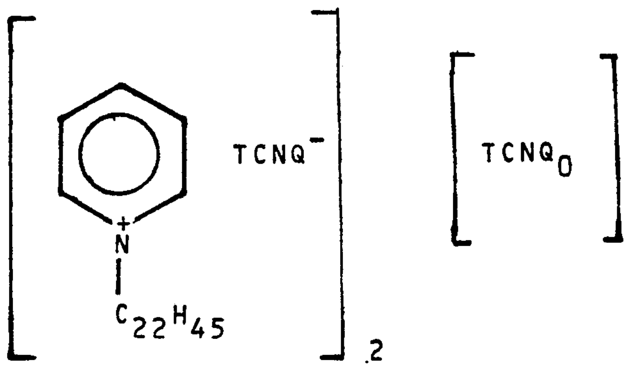

soit un complexe de formule $DA_x$ avec $x=1,5$ et $y=0$.

On traite alors 0,2 mmol du complexe ainsi obtenu par un excés d'iodure de pyridinium dans de l'acétonitrile et l'on obtient un précipité noir conducteur répondant à la formule:

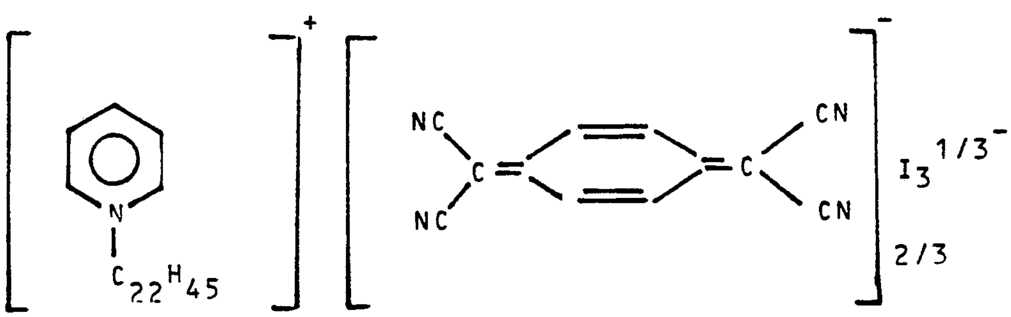

soit un complexe de formule (I) dans laquelle D représente le N-docosylpyridinium, A représente TCNQ, X représente I, $x=2/3$ et $y=3$.

**Exemple 12**

On traite dans de l'acétonitrile 0,3 mmol d'iodure de N-méthylisonicotinate de docosyl par 0,2 mmol de TCNQ. On obtient ainsi un complexe noir de formule:

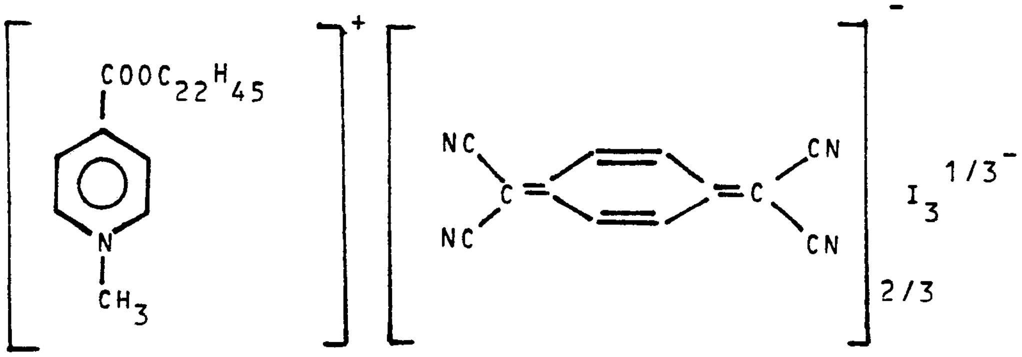

soit un complexe répondant à la formule (I) avec D représentant le N-méthyl isonicotinate de docosyl, A représentant TCNQ, X représentant I, x=2/3 et y=3.

Ce complexe a été caractérisé comme précédemment par spectrophotométrie dans le domaine visible et dans le domaine infrarouge. Des mesures de résistivité ont montré qu'il était conducteur.

**Revendications**

1. Complexe organique amphiphile à transfert de charge, conducteur de l'électricité ou précurseur d'un complexe conducteur de l'électricité, capable d'être déposé en couche monomoléculaire sur un substrat par la méthode de Langmuir-Blodgett, caractérisé en ce qu'il répond à la formule:

$DA_xX_y$ (I)

dans laquelle D représente un groupe monomérique organique donneur d'électrons, A représente un groupe organique accepteur d'électrons, X représente un accepteur d'électrons non amphiphile choisi parmi les acides de Lewis, x est un nombre supérieur à 0 et y est égal à 0 ou est un nombre supérieur à 0, 1,un au moins des groupes D et A étant amphiphile et comportant au moins un substituant hydrocarboné saturé ou insaturé d'au moins 12 atomes de carbone.

2. Complexe selon la revendication 1, caractérisé en ce que X représente un acide de Lewis choisi parmi $PF^-_6$, $ClO^-_4$, $BF^-_4$, $ReO^-_4$, $IO^-_4$, $FSO^-_3$, $AsF^-_6$, $AsF^-_4$, $Br^-$, $Cl^-$, $MnCl^-_6$, les iodures et les oxydes nireux et nitriques.

3. Complexe selon la revendication 1, caractérisé en ce que X représente $I^-$.

4. Complexe selon l'une quelconque des revendications 1 à 3, caractérisé en ce que D représente une base aliphatique, aromatique ou hétérocyclique et A représente un groupe organique choisi parmi le tétracyanoéthylène (TCNE), l'hexacyanobutadiène (HCBD), le 1,2,4,5-tétracyanobenzène (TCNB), le 7,7,8,8-tétracyanoquinodiméthane (TCNQ) et ses dérivés substitués, le tétracyanodiquinodiméthane (TCNDQ), le benzotétracyanoquinodiméthane (benzo-TCNQ), le 2,3,5,6-tétrachloro-p-benzoquinone (CA), le trinitrobenzène (TNB), le tétranaphtoquinodiméthane (TNAP), le thiophénetétracyanoquinodiméthane (thiophène-TCNQ), le sélénophène-tétracyanoquinodiméthane (sélénophène-TCNQ), et le tetracyanoquinoquinazolino quinazoline (TCQQ).

5. Complexe selon la revendication 1, caractérisé en ce que A répond à la formule:

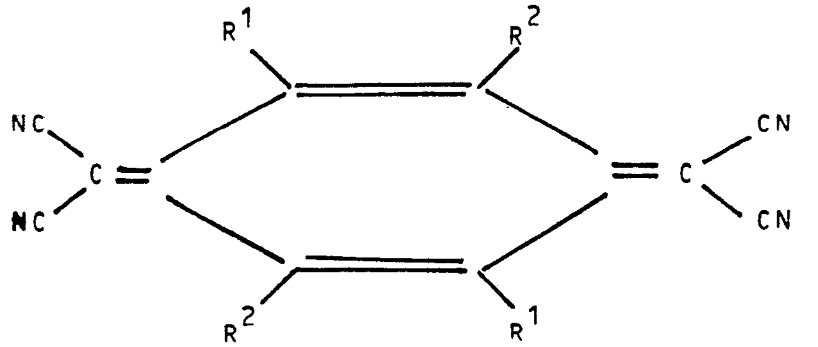

(II)

dans laquelle $R^1$ et $R^2$ qui peuvent être identiques ou différents, représentent un atome d'hydrogène, de chlore, de fluor ou de brome ou un radical alkyle.

6. Complexe selon la revendication 1, caractérisé en ce que y est égal à 0.

7. Complexe selon la revendication 6, caractérisé en ce qu'il répond à la formule suivante:

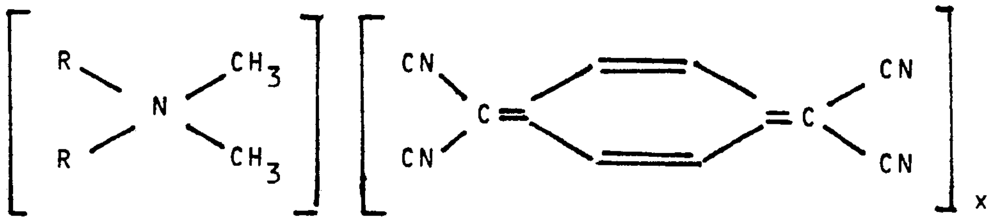

dans laquelle R représente un groupe alkyle de 12 à 30 atomes de carbone et x est égal à 1 ou 1,5.

8. Complexe selon la revendication 6, caractérisé en ce qu'il répond à la formule:

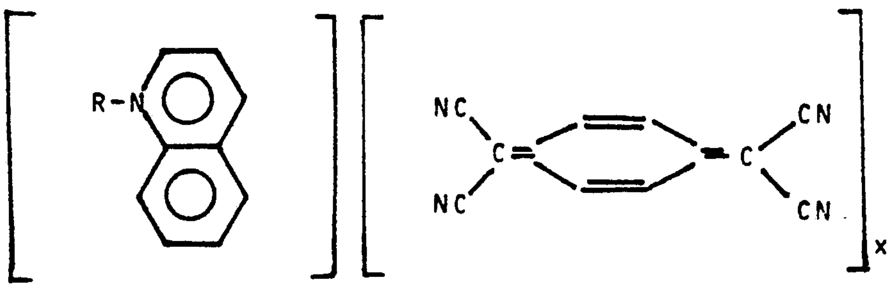

dans laquelle R représente un groupe alkyle de 12 à 30 atomes de carbone et x est égal à 1 ou 1,5.

9. Complexe selon la revendication 6, caractérisé en ce qu'il répond à la formule:

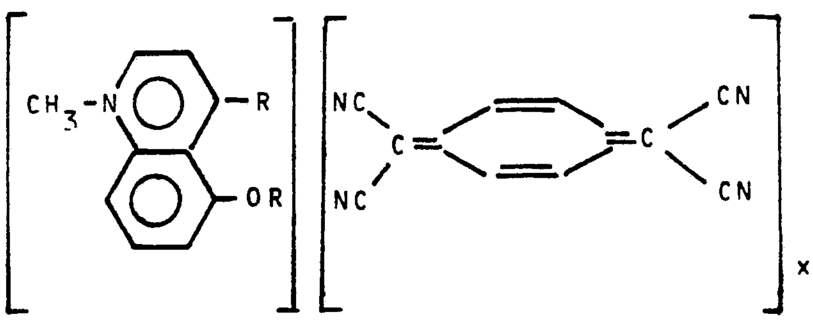

dans laquelle R représente un groupe alkyle de 12 à 30 atomes de carbone et x est égal à 1 ou 1,5.

10. Complexe selon la revendication 6, caractérisé en ce qu'il répond à la formule:

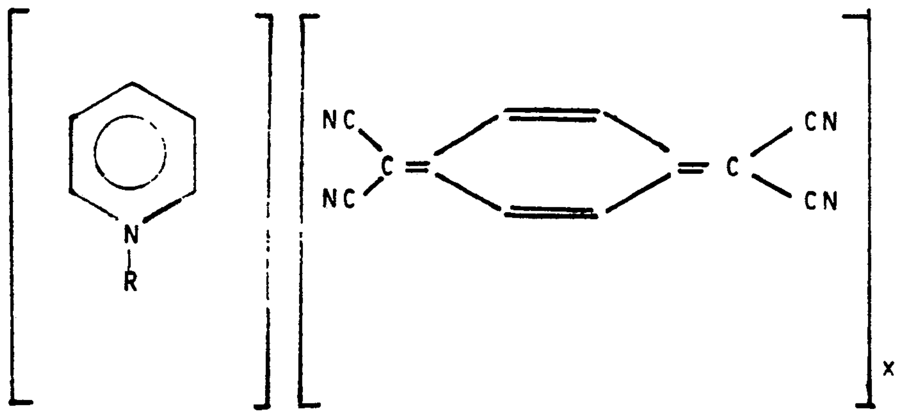

dans laquelle R représente un groupe alkyle de 12 à 30 atomes de carbone et x est égal à 1 ou 1,5.

11. Complexe selon la revendication 6, caractérisé en ce qu'il répond à la formule:

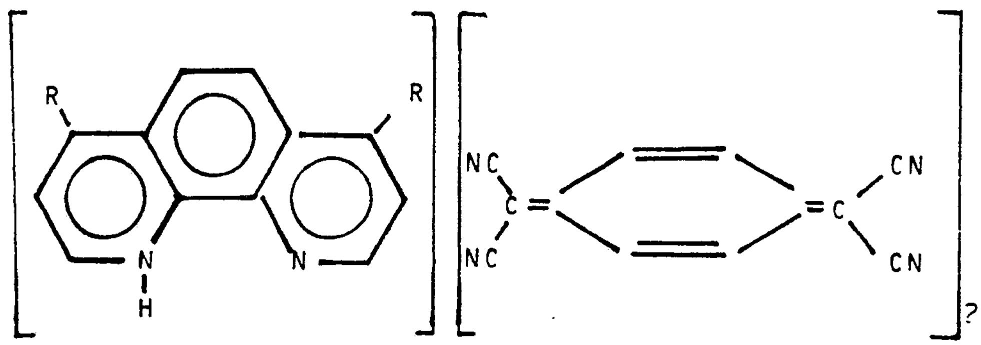

dans laquelle R représente un groupe alkyle de 12 à 30 atomes de carbone.

12. Complexe selon la revendication 6, caractérisé en ce qu'il répond à la formule:

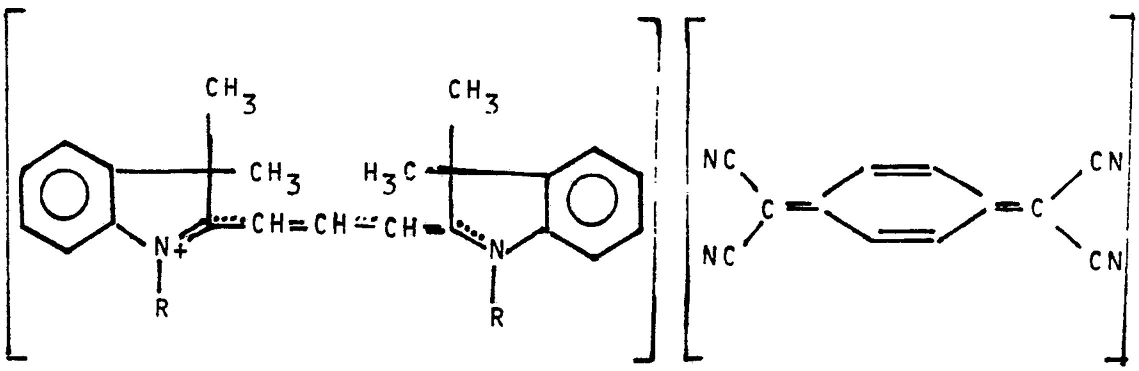

dans laquelle R représente un groupe alkyle de 12 à 30 atomes de carbone.

13. Complexe selon l'une quelconque des revendications 1 à 4, caractérisé en ce que y est un nombre allant de 1 à 20.

14. Complexe selon la revendication 13, caractérisé en ce qu'il répond à la formule:

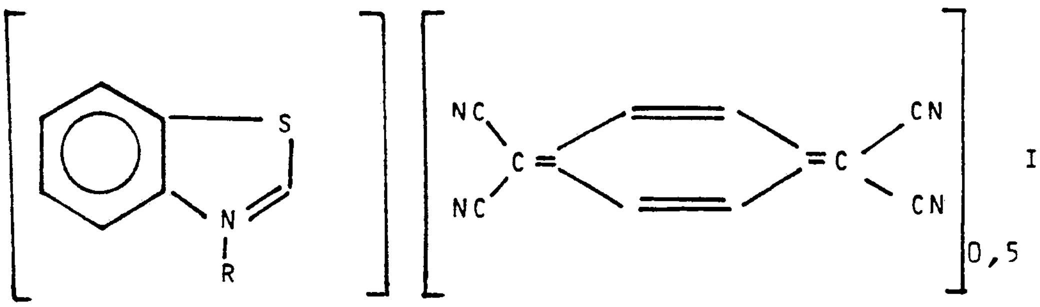

dans laquelle R est un radical alkyle de 12 à 30 atomes de carbone.

15. Complexe selon la revendication 13, caractérisé en ce qu'il répond à la formule:

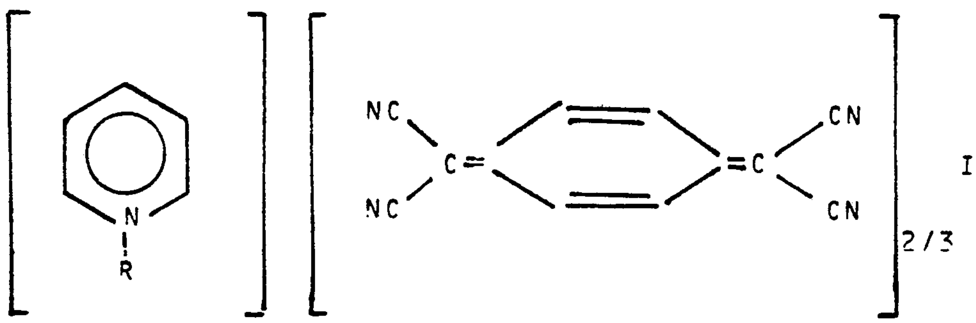

avec R représentant un radical alkyle de 12 à 30 atomes de carbone.

16. Complexe selon la revendication 13, caractérisé en ce qu'il répond à la formule:

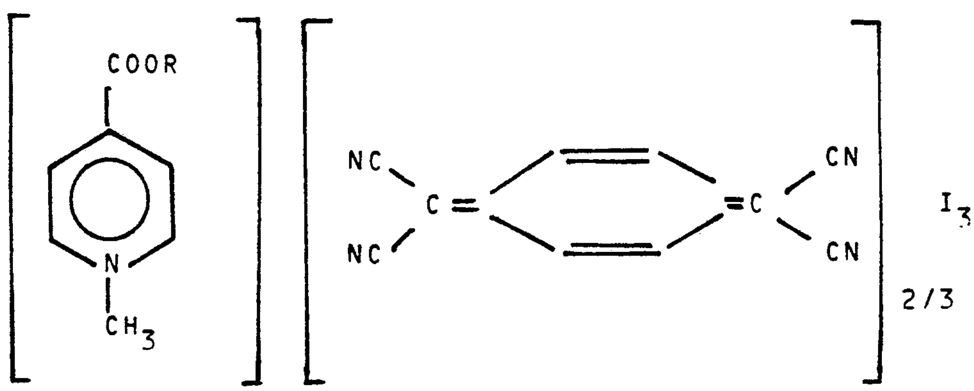

dans laquelle R représente un groupe alkyle de 12 à 30 atomes de carbone.

**Patentansprüche**

1. Elektrisch leitender amphiphiler organischer Komplex für den Ladungstransport oder Vorläufer eines elektrisch leitenden Komplexes, der in Form einer monomolekularen Schicht auf einem Substrat nach dem Langmuir-Blodgett-Verfahren abgeschieden werden kann, dadurch gekennzeichnet, daß er der Formel entspricht:

$DA_xX_y$ (I)

worin bedeuten:

D eine monomere organische Elektronendonor-Gruppe,
A eine organische Elektronenakzeptor-Gruppe,

X einen nicht-amphiphilen Elektronenakzeptor, ausgewählt aus den Lewis-Säuren,
x eine Zahl von größer als 0 und
y die Zahl 0 oder eine Zahl von größer als 0,
wobei mindestens eine der Gruppen D und A amphiphil ist und mindestens einen gesättigten oder ungesättigten Kohlenwasserstoff-Substituenten mit mindestens 12 Kohlenstoffatomen aufweist.

2. Komplex nach Anspruch 1, dadurch gekennzeichnet, daß X eine Lewis-Säure, ausgewählt aus $PF_6^-$, $ClO_4^-$, $BF_4^-$, $ReO_4^-$, $JO_4^-$, $FSO_3^-$, $AsF_6^-$, $AsF_4^-$, $Br^-$, Cl, $MnCl_6^-$, den Jodiden und Oxiden der Salpetrigen Säure und der Salpetersäure, darstellt.

3. Komplex nach Anspruch 1, dadurch gekennzeichnet, daß X für $J^-$ steht.

4. Komplex nach einem der Ansprüche 1 bis 3, dadurch gekennzeichnet, daß D eine aliphatische, aromatische oder heterocyclische Base darstellt und A eine organische Gruppe darstellt, die ausgewählt wird aus Tetracyanoethylen (TCNE), Hexacyanobutadien (HCBD), 1,2,4,5-Tetracyanobenzol (TCNB), 7,7,8,8-Tetracyanochinodimethan (TCNQ) und seinen substituierten Derivaten, Tetracyanodichinodimethan (TCNDQ), Benzotetracyanochinodimethan (Benzo-TCNQ), 2,3,5,6-Tetrachloro-p-benzochinon (CA), Trinitrobenzol (TNB), Tetranaphthochinodimethan (TNAP), Thiophentetracyanochinodimethan (Thiophen-TCNQ), Selenophentetracyanochinodimethan (Selenophen-TCNQ) und Tetracyanochinochinazolinochinazolin (TCQQ).

5. Komplex nach Anspruch 1, dadurch gekennzeichnet, daß A der Formel entspricht:

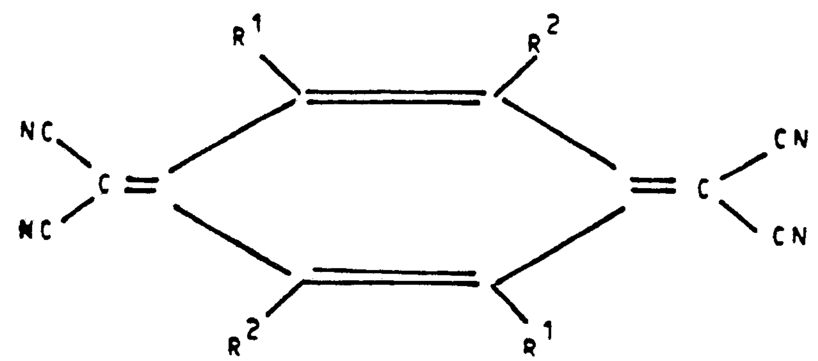

(II)

worin $R^1$ und $R^2$, die gleich oder verschieden sein können, ein Wasserstoff-, Chlor-, Fluor- oder Bromatom oder einen Alkylrest bedeuten.

6. Komplex nach Anspruch 1, dadurch gekennzeichnet, daß y die Zahl 0 darstellt.

7. Komplex nach Anspruch 6, dadurch gekennzeichnet, daß er der folgenden Formel entspricht:

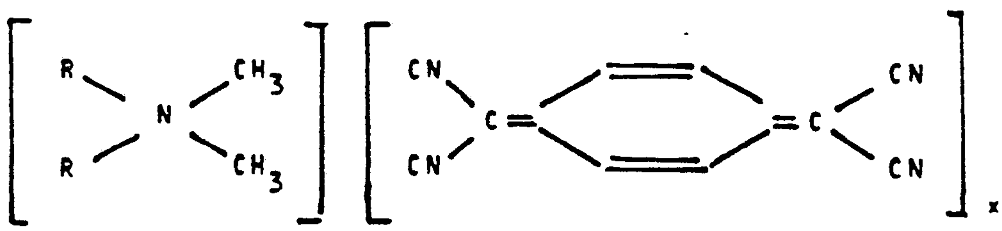

worin R eine Alkylgruppe mit 12 bis 30 Kohlenstoffatomen und x die Zahl 1 oder 1,5 darstellen.

8. Komplex nach Anspruch 6, dadurch gekennzeichnet, daß er der folgenden Formel entspricht:

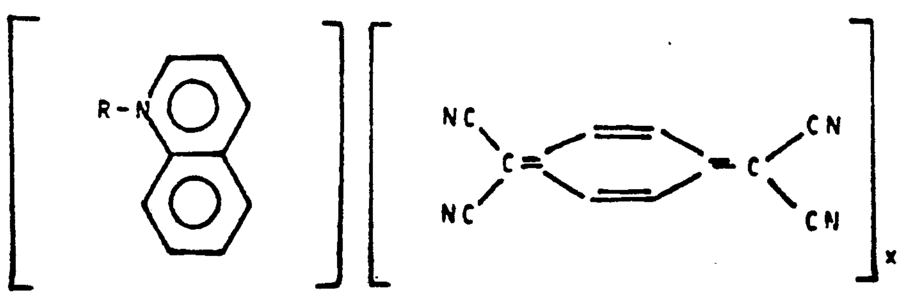

worin R eine Alkylgruppe mit 12 bis 30 Kohlenstoffatomen und x die Zahl 1 oder 1,5 darstellen.

9. Komplex nach Anspruch 6, dadurch gekennzeichnet, daß er der Formel entspricht:

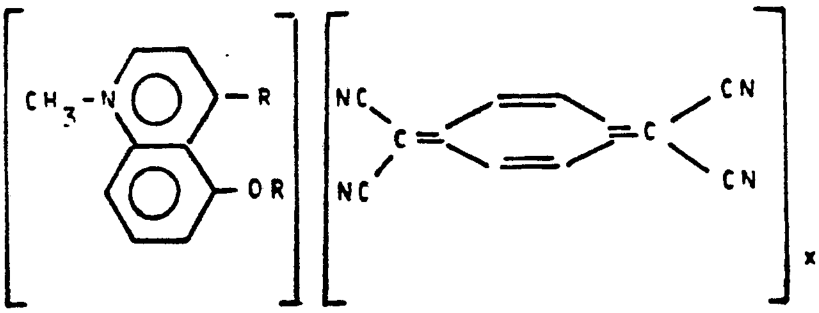

worin R eine Alkylgruppe mit 12 bis 30 Kohlenstoffatomen und x die Zahl 1 oder 1,5 darstellen.

10. Komplex nach Anspruch 6, dadurch gekennzeichnet, daß er der Formel entspricht:

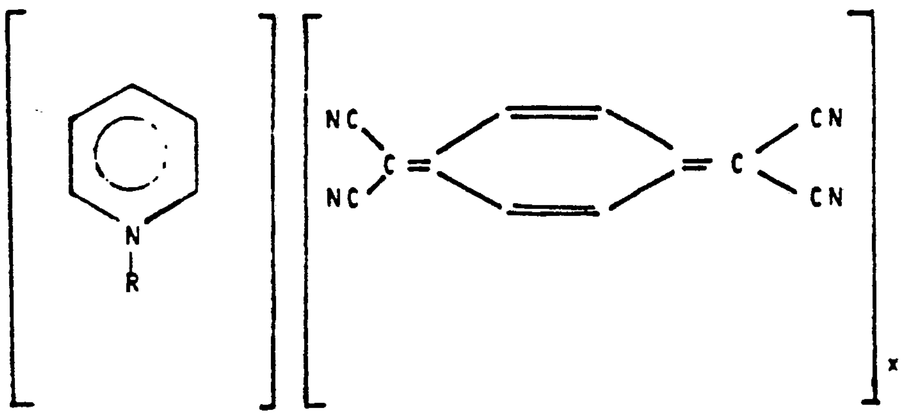

worin R eine Alkylgruppe mit 12 bis 30 Kohlenstoffatomen und x die Zahl 1 oder 1,5 darstellen.

11. Komplex nach Anspruch 6, dadurch gekennzeichnet, daß er der Formel entspricht:

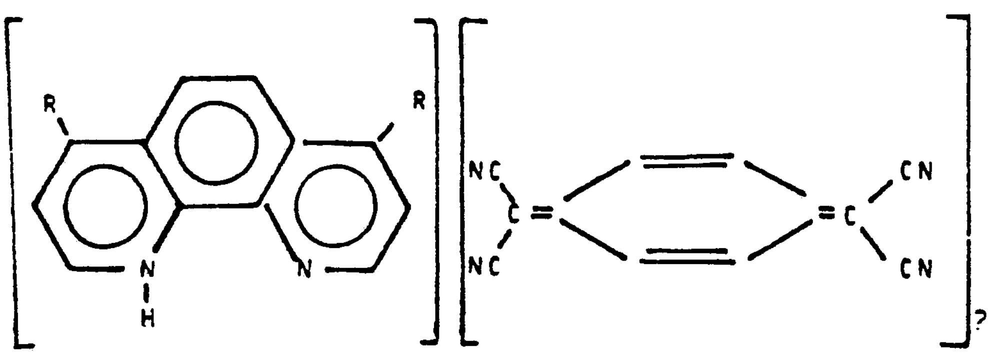

worin R eine Alkylgruppe mit 12 bis 30 Kohlenstoffatomen darstellt.

12. Komplex nach Anspruch 6, dadurch gekennzeichnet, daß er der Formel entspricht:

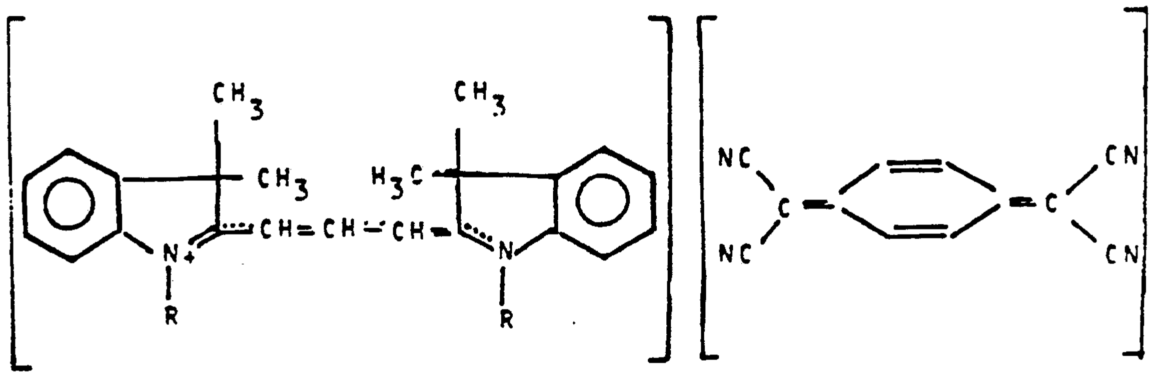

worin R eine Alkylgruppe mit 12 bis 30 Kohlenstoffatomen darstellt.

13. Komplex nach einem der Ansprüche 1 bis 4, dadurch gekennzeichnet, daß y für eine Zahl von 1 bis 20 steht.

14. Komplex nach Anspruch 13, dadurch gekennzeichnet, daß er der Formel entspricht

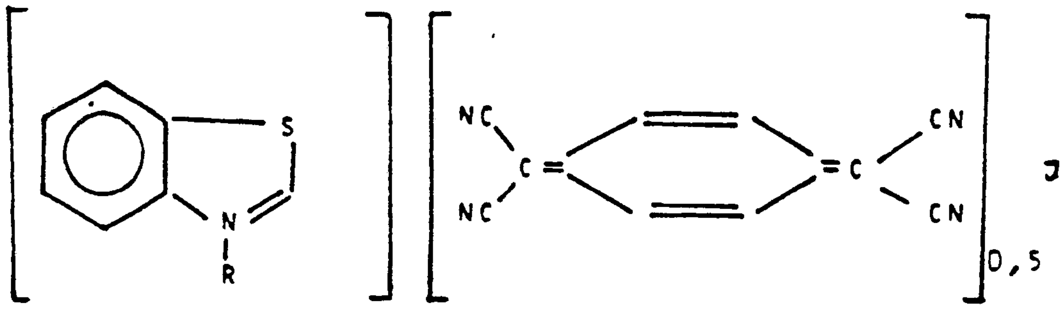

worin R einen Alkylrest mit 12 bis 30 Kohlenstoffatomen darstellt.

15. Komplex nach Anspruch 13, dadurch gekennzeichnet, daß er der Formel entspricht:

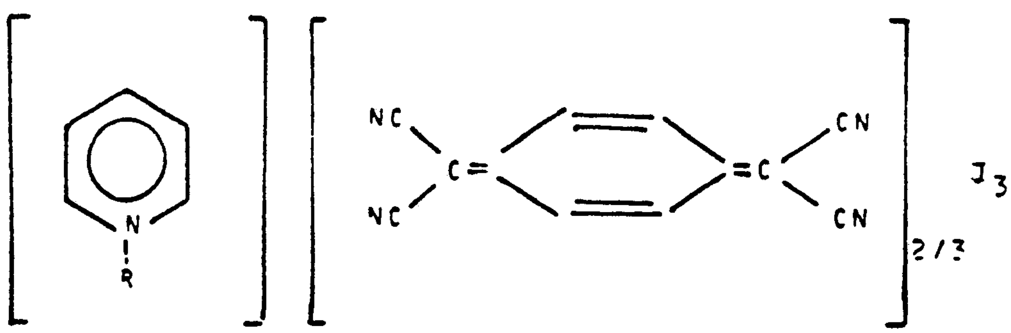

worin R einen Alkylrest mit 12 bis 30 Kohlenstoffatomen darstellt.

16. Komplex nach Anspruch 13, dadurch gekennzeichnet, daß er der Formel entspricht:

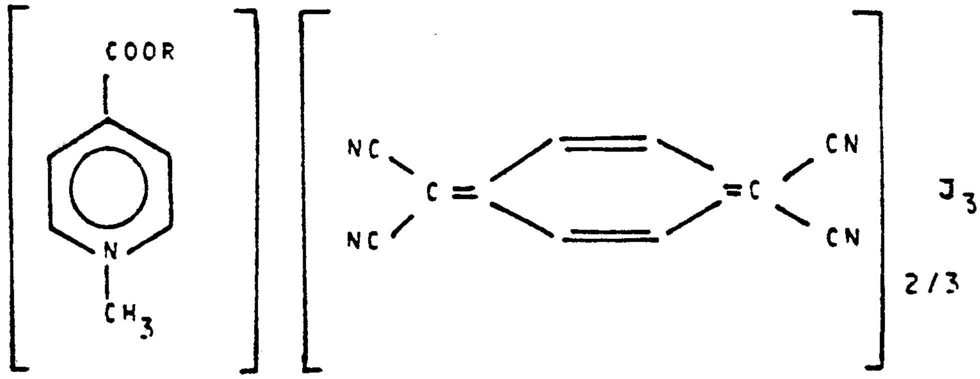

worin R eine Alkylgruppe mit 12 bis 30 Kohlenstoffatomen darstellt.

## Claims

1. Organic amphiphilic charge transfer complex, which is an electrical conductor or a precursor of an electrical conductor complex, which can be deposited as a monomolecular layer on a substrate by the Langmuir-Blodgett method, characterized in that it complies with formula:

$DA_xX_y$ (I)

in which D represents a monomeric organic electron donor group, A represents an organic electron acceptor group, X represents a non-amphiphilic electron acceptor chose, from among the Lewis acids, x is a number greater than 0 and y is equal to 0 or is a number greater than 0, at least one of the groups 0 and A being amphiphilic and having at least one saturated or unsaturated hydrocarbon substituent with at least 12 carbon atoms.

2. Complex according to claim 1, characterized in that X represents a Lewis acid chosen from among $PF^-_8$, $ClO^-_4$, $BF^-_4$, $REO^-_4$, $IO^-_4$, $FSO^-_3$, $AsF^-_6$, $AsF^-_4$, $Br^-$, $Cl^-$, $MnCl^-_6$, iodides, nitrous oxides and nitric oxides.

3. Complex according to claim 1, characterized in that X represents $I^-$.

4. Complex according to any one of the claims 1 to 3, characterized in that D represents an aliphatic, aromatic or heterocyclic base and A represents an organic group chosen from among tetracyanoethylene (TCNE), hexacyanobutadiene (HCBD) 1,2,4,5-tetracyanobenzene (TCNB), 7,7,8,8-tetracyanoquinodimethane (TCNQ) and its derivatives, tetracyanodiquinodimethane (TCNDQ), benzotetracyanoquinodimethane (benzo-TCNQ), 2,3,5,8-tetrachloro-p-benzoquinone (CA), trinitrobenzene (TNB), tetranaphthoquinodimethane (TNAP), thiophenetetracyanoquinodimethane (thiophene-TCNQ), selenophene-tetracyanoquinodimethane (selenophene-TCNQ), and tetracyanoquinoquinazolino-quinazoline (TCQQ).

5. Complex according to claim 1, characterized in that A is in accordance with formula:

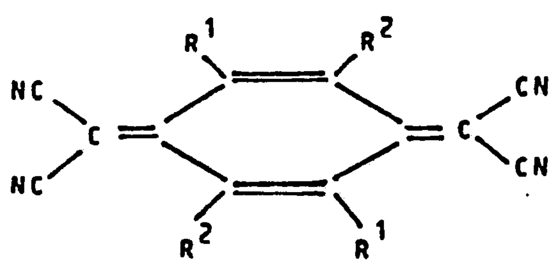

(II)

in which $R^1$ and $R^2$ which can be the same or different, represent a hydrogen, chlorine, fluorine or bromine atom or an alkyl radical.

8. Complex according to claim 1, characterized in that y is equal to 0.

7. Complex according to claim 8, characterized in that it is in accordance with the following formula:

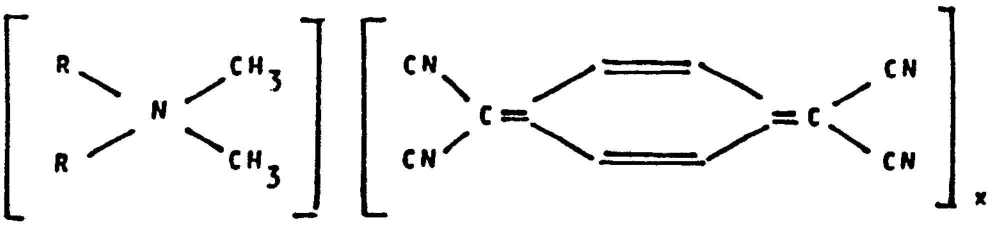

in which R represents an alkyl group with 12 to 30 carbon atoms and x is equal to 1 or 1.5.

8. Complex according to claim 8, characterized in that it is in accordance with the formula:

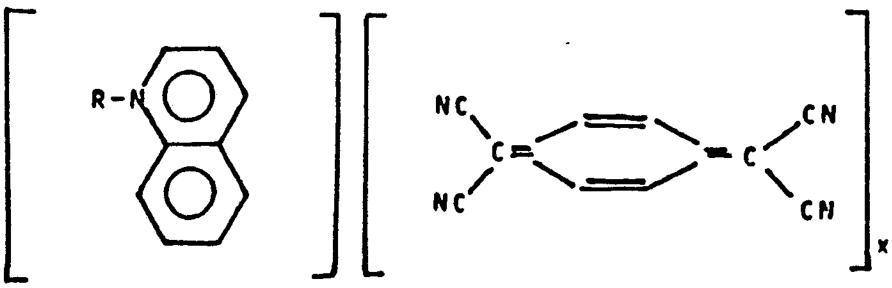

in which R represents an alkyl group with 12 to 30 carbon atoms and x is equal to 1 or 1.5.

9. Complex according to claim 8, characterized in that it is in accordance with the formula:

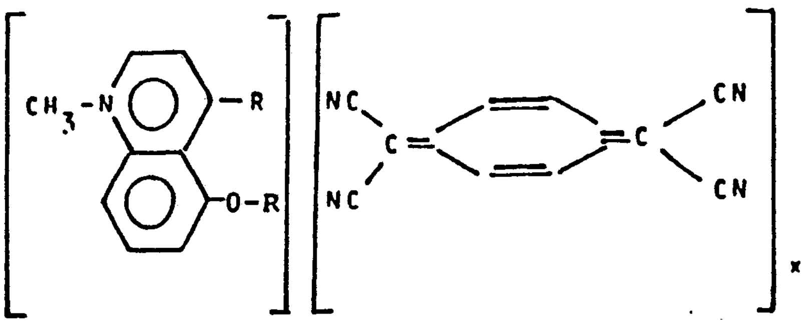

in which R represents an alkyl group with 12 to 30 carbon atoms and x is equal to 1 or 1.5.

10. Complex according to claim 8, characterized that it is in accordance with the formula:

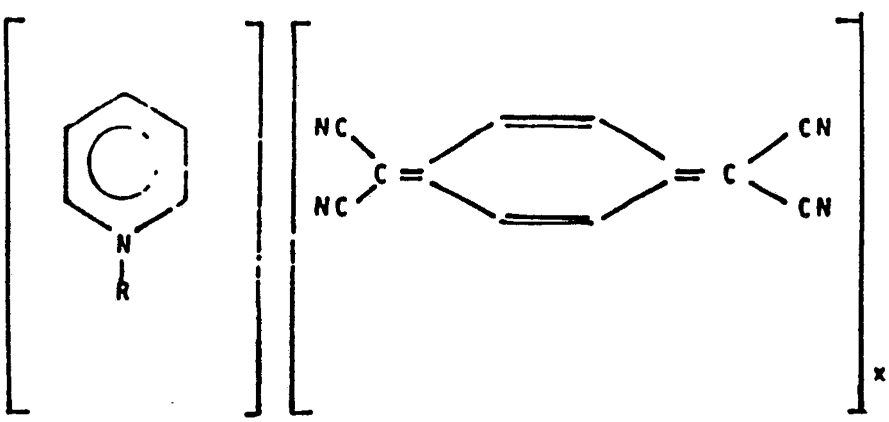

in which R represents an alkyl group with 12 to 30 carbon atoms and x is equal to 1 or 1.5.

11. Complex according to claim 8, characterized in that it is in accordance with the formula:

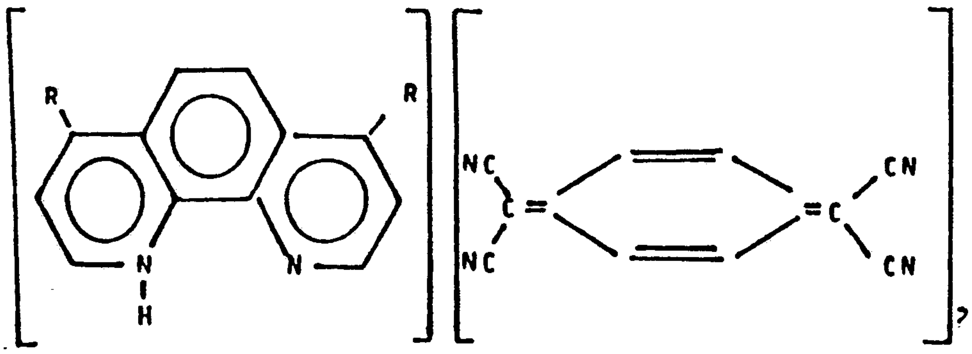

in which R represents an alkyl group with 12 to 30 carbon atoms.

12. Complex according to claim 6, characterized in that it is in accordance with the formula:

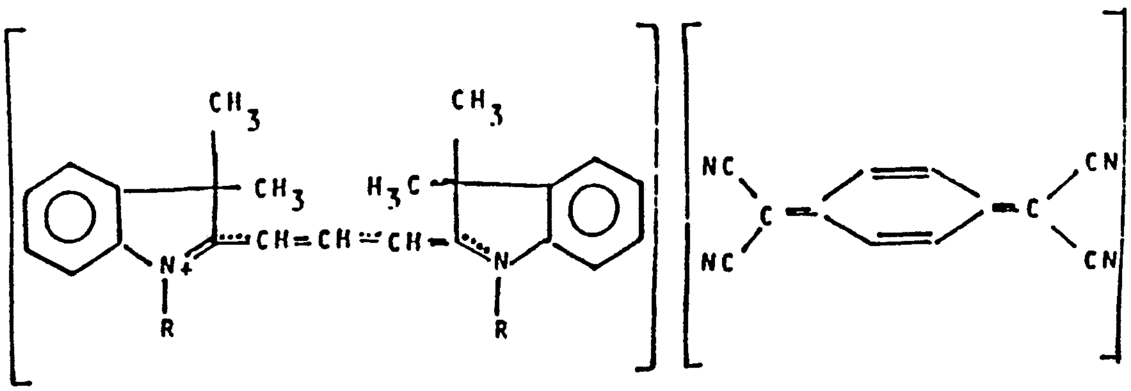

in which R represents an alkyl group with 12 to 30 carbon atoms.

13. Complex according to any one of the claims 1 to 4, characterized in that y is a number between 1 and 20.

14. Complex according to claim 13, characterized in that it is in accordance with the formula:

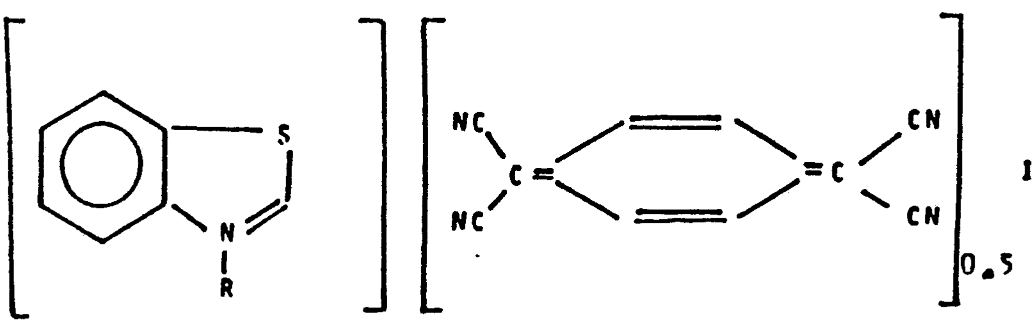

in which R is an alkyl radical with 12 to 30 carbon atoms.

15. Complex according to claim 13, characterized in that it is in accordance with the formula:

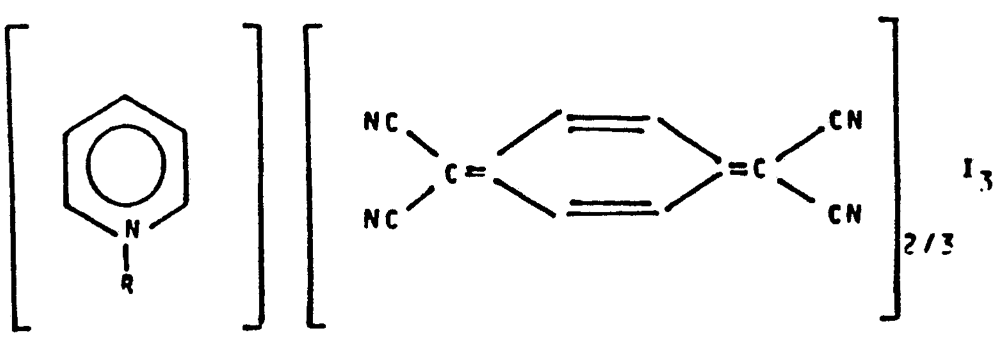

in which R represents an alkyl radical with 12 to 30 carbon atoms.

16. Complex according to claim 13, characterized in that it is in accordance with the formula:

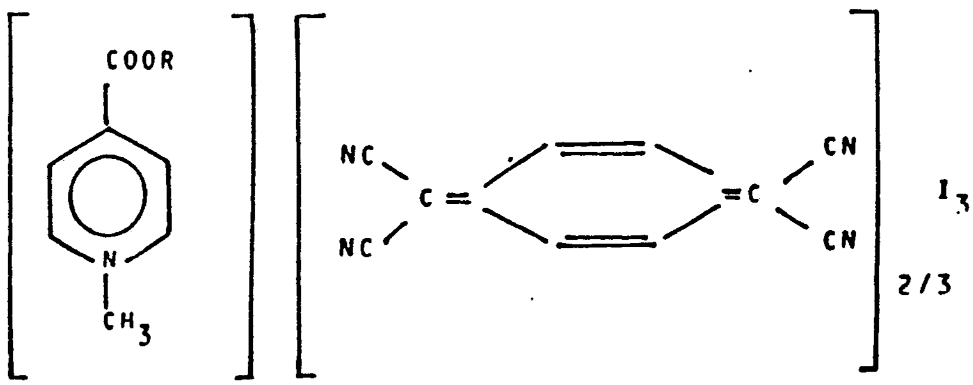

in which R represents an alkyl group with 12 to 30 carbon atoms.